(19) Europäisches Patentamt
European Patent Office
Office européen des brevets

(11) Publication number: 0 160 258 B1

(12) EUROPEAN PATENT SPECIFICATION

(45) Date of publication of patent specification: 27.09.89

(51) Int. Cl.$^4$: C 07 D 313/12, C 07 C 69/76, A 61 K 31/335

(21) Application number: 85104830.6

(22) Date of filing: 20.04.85

# (54) (6,11-Dihydro-11-oxodibenz(b,e)oxepinyl)pentanols, a process and intermediates for their preparation and their use as medicaments.

(30) Priority: 27.04.84 US 604545

(43) Date of publication of application: 06.11.85 Bulletin 85/45

(45) Publication of the grant of the patent: 27.09.89 Bulletin 89/39

(84) Designated Contracting States: AT BE CH DE FR GB IT LI LU NL SE

(56) References cited:
DE-A-2 435 613

PATENTS ABSTRACTS OF JAPAN, vol. 4, no. 186, (C-36)668r, 20th December 1980; & JP - A - 55 124 777 (TOUA EIYOU KAGAKU KOGYO K.K.) 26.09.1980

(73) Proprietor: HOECHST-ROUSSEL PHARMACEUTICALS INCORPORATED
Route 202-206 North
Somerville New Jersey 08876 (US)

(72) Inventor: Martin, Lawrence L.
R.D. 3, Concord Road Box 81
Lebanon New Jersey 08833 (US)
Inventor: Setescak, Linda L.
Kimberly Road 102
Somerville New Jersey 08876 (US)

(74) Representative: Meyer-Dulheuer, Karl-Hermann, Dr. et al
HOECHST Aktiengesellschaft Postfach 3540
D-6121 Wiesbaden (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 160 258 B1

Courier Press, Leamington Spa, England.

## Description

The present invention relates to (6,11-dihydro-11-oxodibenz[b,e]oxepinyl)pentanols and derivatives thereof. More particular, the present invention relates to (6,11-dihydro-11-oxodibenz[b,e]oxepinyl)-pentanols of formula *1*

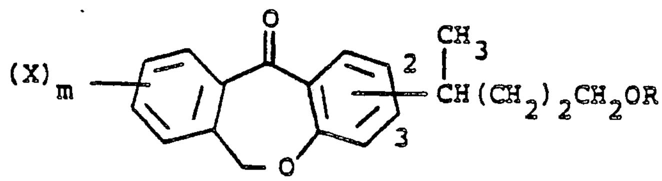 1

wherein the group

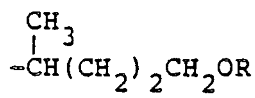

is bound to the 2- or 3-position and wherein R is hydrogen or a group of the formula

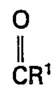

wherein $R^1$ is hydrogen, alkyl of 1 to 5 carbon atoms, benzyl, phenyl or phenyl substituted by alkyl of 1 to 5 carbon atoms, alkoxy of 1 to 5 carbon atoms, halogen or trifluoromethyl; X is alkyl of 1 to 5 carbon atoms, alkoxy of 1 to 5 carbon atoms, halogen or trifluoromethyl; m is 0, 1 or 2, or the optical antipode thereof, which are useful as antiarthritic agents, alone or in combination with inert pharmaceutical carriers.

DE—A—24 35 613 discloses 2-(hydroxyalkyl)-6,11-dihydro-11-oxodibenz[b,e]-oxepines having analgesic, antipyretic and anti-inflammatory activity.

JP—A—55/124 777 (Pat. Abstr. of Japan, Vol. 4, No. 186 (C—36) [668], 1980) describes 2-alkyl-6,11-dihydro-11-oxodibenz[b,e]oxepin derivatives, in which the 2-alkyl-group may be substituted with hydroxy, having anti-inflammatory activity.

Preferred (6,11-dihydro-11-oxodibenz[b,e]oxepinyl)pentanols and derivatives thereof of the present invention are those wherein R is hydrogen and m is 0.

The present invention also relates to [(2-carboxybenzyloxy)phenyl]pentanols and derivatives thereof of formula *2*

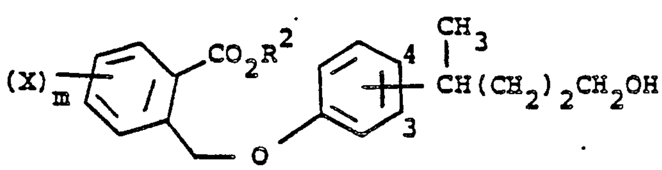 2

wherein the group

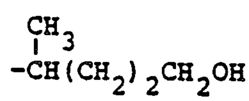

is bound to either the 3- or 4-position, and wherein $R^2$ is hydrogen or alkyl of 1 to 5 carbon atoms; X is alkyl of 1 to 5 carbon atoms, alkoxy of 1 to 5 carbon atoms, halogen or trifluoromethyl; m is 0, 1 or 2, or an optical antipode thereof, and (hydroxyphenyl)pentanols of formula *3*

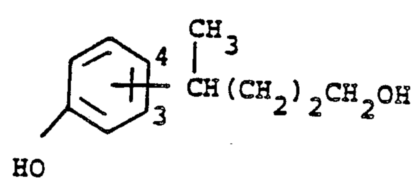 3

wherein the group

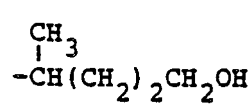

is bound to either the 3- or 4-position, or an optical antipode thereof, useful as intermediates for the preparation of the hereinbeforementioned (6,11-dihydro-11-oxodibenz[b,e]oxepinyl)pentanols and derivatives thereof.

Preferred [(2-carboxybenzyloxy)phenyl]pentanols and derivatives thereof are those wherein $R^2$ is hydrogen or alkyl of 1 to 5 carbon atoms, and m is 0.

As used through the specification and appended claims, the term "alkyl" refers to a straight or branched chain hydrocarbon radical containing no unsaturation and having 1 to 5 carbon atoms such as methyl, ethyl, 1-propyl, 2-propyl, 1-butyl, 1-pentyl, 2-pentyl; the term "alkoxy" refers to monovalent substituent which consists of an alkyl group linked through an ether oxygen and having its free valence bond from the ether oxygen such as methoxy, ethoxy, propoxy, butoxy, 1,1-dimethylethoxy, pentoxy; the terms "alkanoic acid" refers to a compound formed by combination of a carboxyl group with a hydrogen atom or alkyl group. Examples of alkanoic acids are formic acid, acetic acid, propanoic acid, 2,2-dimethylacetic acid, pentanoic acid; the term "halogen" refers to a member of the family fluorine, chlorine, bromine or iodine.

The compounds of the present invention which lack an element of symmetry exist as optical antipodes and as the racemic forms thereof. The optical antipodes may be prepared from the corresponding racemic forms by standard optical resolution techniques, or by synthesis from optically active precursors.

The present invention comprehends all optical isomers and racemic forms thereof of the compounds disclosed and claimed herein. The formulas of the compounds shown herein are intended to encompass all possible optical isomers of the compounds so depicted.

The novel (6,11-dihydro-11-oxodibenz[b,e]oxepinyl)pentanols *1* of the present invention are synthesized by the processes depicted in Reaction Scheme A.

Condensation of a benzylhaloester *4* with a phenolic pentanol *3* provides a benzyloxyester *2* which is hydrolyzed to a benzyloxyacid *2a* and, in turn, cyclized to a (6,11-dihydro-11-oxodibenz[b,e]-oxepinyl)pentanol *1a*. The pentanol *1a* may be esterified to a compound of formula *1*.

The condensation is accomplished by contacting a benzylhaloester *4* with a phenolic alkanol *3* in an alkanone in the presence of an alkali metal carbonate. Among alkanones there may be mentioned 2-propanone, 2-butanone and 3-pentanone. Among alkali metal carbonates there may be mentioned lithium, sodium and potassium carbonate. 2-Butanone and potassium carbonate are the preferred alkanone and alkali metal carbonate, respectively. The temperature at which the condensation is conducted is not critical. However, to ensure a reasonable rate of reaction, the condensation is preferably performed at the reflux temperature of the reaction medium.

A condensation promoter such as an alkali metal halide, for example, lithium, sodium or potassium bromide, or lithium, sodium or potassium iodide, may be employed. Potassium iodide is the preferred promoter.

The hydrolysis is effected by treating an ester *2* with an alkali metal hydroxide such as lithium hydroxide, sodium hydroxide or potassium hydroxide in an aqueous alkanol such as methanol, ethanol, 2-propanol, butanol, pentanol. Potassium hydroxide and aqueous ethanol comprise the preferred reaction medium. The hydrolysis proceeds at ordinary temperatures. To facilitate the reaction, however, elevated temperatures falling within the range of ambient temperature to the reflux temperature of the reaction medium may be employed. The reflux temperature of the reaction medium is preferred.

The cyclization is preferably conducted by contacting an acid *2a* with trifluoroacetic anhydride in a halocarbon solvent, such as dichloromethane, trichloromethane, tetrachloromethane, 1,1-dichloromethane or 1,2-dichloroethane.

The cyclization temperature is not narrowly critical. The reaction proceeds at ambient temperature (25°C) to the reflux temperature of the reaction medium. The reflux temperature of the reaction medium is preferred. Under these conditions, the trifluoroacetyl derivative *1* wherein R is

$$\overset{O}{\overset{\|}{C}}CF_3$$

is obtained. The trifluoroacetyl group is readily removed by hydrolysis to afford *1a*.

The cyclization proceeds efficiently when two or more molar-equivalents of trifluoroacetic anhydride (relative to an acid 2a) are employed. It is preferable to conduct the cyclization employing about two to about six molar-equivalents of trifluoroacetic anhydride, and most preferable to employ about three molar equivalents of the cyclizing agent.

The cyclization of an acid *2a* to an oxepinonepentanol *1a* may be effected by means of phosphorus-containing agents such as phosphorus pentoxide, phosphorus pentoxide-alkanol, polyphosphoric acid, polyphosphoric acid-alkanoic acid and the like. For example, treatment of an acid *2a* with phosphorus pentoxide or phosphorus pentoxide-ethanol in the presence or absence of a solvent, such as sulfolane, at ambient to moderate temperatures within the range of about 25° to 120°C, (a cyclization temperature of about 90°C being preferred), and a treatment of an acid *2a* with polyphosphoric acid or polyphosphoric acid-acetic acid at ambient to moderate temperatures within the range of about 25° to 100°C, (a reaction temperature of about 80°C being preferred), affords oxepinone *1a*.

A (6,11-dihydro-11-oxodibenz[b,e]oxepinyl)pentanol *1a* may also be converted to the corresponding ester *1* wherein R is

$$\overset{O}{\overset{\|}{C}}R^1$$

wherein $R^1$ is as hereinbeforedescribed by techniques, involving, for example, treatment of a pentanol *1a* with a carboxylic acid, such as, for example, an alkanoic acid, or benzyl- or phenylcarboxylic acid in the presence of a mineral acid such as sulfuric acid, hydrochloric acid and the like, or a strong organic acid such as methanesulfonic acid or 4-methylphenylsulfonic acid.

Benzylhaloester *4* is prepared by procedures described and referenced in D. E. Aultz, et al., J. Med. Chem., *20*, 66 (1977).

Phenolic pentanol *3* is prepared by the sequence depicted in Reaction Scheme B, involving esterification of nitrophenylacid *5*, the synthesis of which is described in J. Colonge and E. Fichet, Bull. Soc. Chem. France, 412 (1955), to nitrophenylester *6*, followed by reduction of nitrophenylester *6* to aminophenylester *7*, conversion of aminophenylester *7* to hydroxyphenylester *8*, and reduction of hydroxyphenylester *8* to hydroxyphenylalkanol *3*. The transformation of nitrophenylacid *5* to hydroxyphenylester *8* is carried through by esterification of the acid *5* to the alkyl nitrophenylpentenoate *6*, followed by reduction of this compound to alkyl aminophenylpentenoate *7* and conversion of this compound to alkyl hydroxyphenylpentenoate *8*. Thus, treatment of acid *5* with an alkanol, e.g. ethanol, in the presence of a mineral acid, e.g., sulfuric acid, provides ester *6* which is reduced to aniline *7* with hydrogen in the presence of hydrogenation catalyst, e.g., palladium-on-carbon, and, in turn, diazotized with nitrous acid, generated *in situ* from an alkali metal nitrite, e.g., sodium nitrite, and a mineral acid, e.g., sulfuric acid, to a diazonium salt and, without isolation, hydrolyzed to the phenol *8* by means of aqueous copper sulfate solution.

The reduction of hydroxyphenylester *8* to hydroxyphenylpentanol *3* is effected by means of a borane alkyl sulfide/dichloromethane in a suitable solvent. Among borane alkyl sulfides/dichloromethanes, there may be mentioned borane methyl sulfide/dichloromethane, borane ethyl sulfide/dichloromethane and the like. Borane methyl sulfide/dichloromethane is preferred. Among suitable solvents, there may be mentioned aromatic solvents such as benzene, toluene, xylene and the like. Toluene is preferred. The reduction temperature is not critical. To assure a reasonable rate of reduction, however, the reaction may be conducted at an elevated temperature of about the reflux temperature of the medium.

The (6,11-dihydro-11-oxodibenz[b,e]oxepinyl)pentanols and derivatives thereof of the present invention are useful as antiarthritic agents due to their ability to specifically suppress arthritis-type inflammation in mammals. The antiarthritic utility is demonstrated in the adjuvant-induced polyarthritis syndrome in rats, by a procedure similar to that described by C. M. Perrone and F. D. Wood, Arthritis and Rheumatism, *2*, 440 (1959).

Groups of 10 male Charles River-Wistar Lewis rats weighing 150 to 175 g were individually housed and maintained on a regular rat chow diet. Water was given *ad libitum.* The adjuvant was prepared by suspending 75 mg of *Mycobacterium butyricum* (Difeo Laboratories, Detroit, Michigan) in 10 ml of white paraffin oil with continuous stirring for 2 hrs at room temperature prior to administration. Test compounds are prepared by suspending the drug in water, adding one drop of Tween 80 per 10 ml of suspension, and homogenizing. The adjuvant suspension (0.1 ml) was injected into the footpad of the left hind paw of the rat. Test compound suspensions were administered orally (10 ml/kg) the day before adjuvant suspension injection and the administration was continued for 21 days. One group of ten rats was used for the test drug, standard, adjuvant-injected control and non-injected control. Control animals received vehicle (10 ml/kg). Three doses of test drug and one dose of standard preparation were used. Injected and non-injected paw volumes were determined on the day the adjuvant suspension was given, and on subsequent days thereafter (usually days 5, 10, 18, and 21) by the method of C. A. Wilson, et al., Proc. Soc. Exp. Biol. Med., *111*, 544 (1962).

The percent inhibition of paw volume (injected and non-injected hind paw) was calculated by the following formula:

$$\%\ \text{Inhibition} = \frac{\begin{array}{c}\text{Mean Paw Volume} \\ \text{Change of Injected} \\ \text{(or Non-injected) Control}\end{array} - \begin{array}{c}\text{Mean Paw Volume} \\ \text{Change of Drug} \\ \text{Treated}\end{array}}{\text{Mean Paw Volume Change of Injected (or Non-injected) Control)}} \times 100$$

$ED_{50}$-values, i.e., the dose at which the drug effects a 50% inhibition of paw volume, were estimated by the method of J. T. Litchfield and F. Wilcoxon, J. Pharm. Exp. Thes., *96*, 99 (1948) and statistically evaluated by means of the student "t" test. $ED_{50}$-values of a compound of the present invention and a standard are presented below:

| Compound | $ED_{50}$ (mg/kg/day) |
|---|---|
| 4-(6,11-dihydro-11-oxodibenz[b,e]oxepin-2-yl)pentanol | 5.6 (injected)<br>3.9 (non-injected) |
| phenylbutazone | 12.0 (injected)<br>6.8 (non-injected) |

Arthritic-like inflammation suppression is achieved when the present (6,11-dihydro-11-oxidibenz[b,e]-oxepinyl)pentanols are administered to a subject requiring such treatment as an effective oral, parenteral or intravenous dose of from 0.01 to 100 mg/kg of body weight per day. A particularly effective amount is about 25 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only and they do not, to any extent, limit the scope of practice of the invention.

Effective amounts of a compound of the present invention may be administered to a subject by any one of various methods, for example, orally as in capsules or tablets, parenterally in the form of sterile solutions or suspensions, and in some cases intravenously in the form of sterile solutions.

Effective quantities of the compounds of the invention may be administered orally for example, with an inert diluent or with an edible carrier. They may be enclosed in gelatin capsules or compressed into tablets. For the purpose of oral therapeutic administration, the aforesaid compounds may be incorporated with excipient and used in the form of tablets, troches, capsules, elixirs, suspensions, syrups, wafers chewing gums and the like. These preparations should contain at least 0.5% of active compound, but may be varied depending upon the particular form and may conveniently be between 4% to about 70% of the weight of the unit. The amount of active compound in such composition is such that a suitable dosage will be obtained. Preferred compositions and preparations according to the present invention are prepared so that an oral dosage unit form contains between 1.0—300 milligrams of the active compound.

The tablets, pills, capsules, troches and the like may also contain the following ingredients: a binder such as microcrystalline cellulose, gum tragancanth or gelatin; an excipient such as starch or lactose, a disintegrating agent such as alginic acid, corn starch and the like; a lubricant such as magnesium stearate; a glidant such as colloidal silicon dioxide; and a sweetening agent such as sucrose or saccharin or a flavoring agent such as peppermint, methyl salicylate, or orange flavoring may be added. When the dosage unit form is a capsule, it may contain, in addition to materials of the above type, a liquid carrier such as a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purposes of parenteral therapeutic administration, the active compounds of the invention may be incorporated into a solution or suspension. These preparations should contain at least 0.1% of active compound, but may be varied between 0.5 and about 50% of the weight thereof. The amount of active compound in such compositions is such that a suitable dosage will be obtained. Preferred compositions and preparations according to the present invention are prepared so that a parenteral dosage unit contains between 0.5 to 100 milligrams of active compound.

The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

While the (6,11-dihydro-11-oxodibenz[b,e]oxepinyl)pentanols of the present invention exhibit antiarthritic activity in the adjuvant-induced polyarthritis syndrone assay as hereinbefore discussed, the present compounds are essentially inactive antiinflammatories in an art-recognized assay for antiinflammatory activity, the carrageenan paw assay (D. E. Aultz, et al., J. Med. Chem., *20*, 66 (1977)).

The (6,11-dihydro-11-oxodibenz[b,e]oxepinyl)pentanols of the present invention are also essentially inactive analgetics in an art-recognized assay for analgesia, the phenyl-parabenzoquinone assay for analgetic activity (E. Siegmund, et al., Proc. Soc., Explt. Biol. Med., *95*, 729 (1957).

REACTION SCHEME A

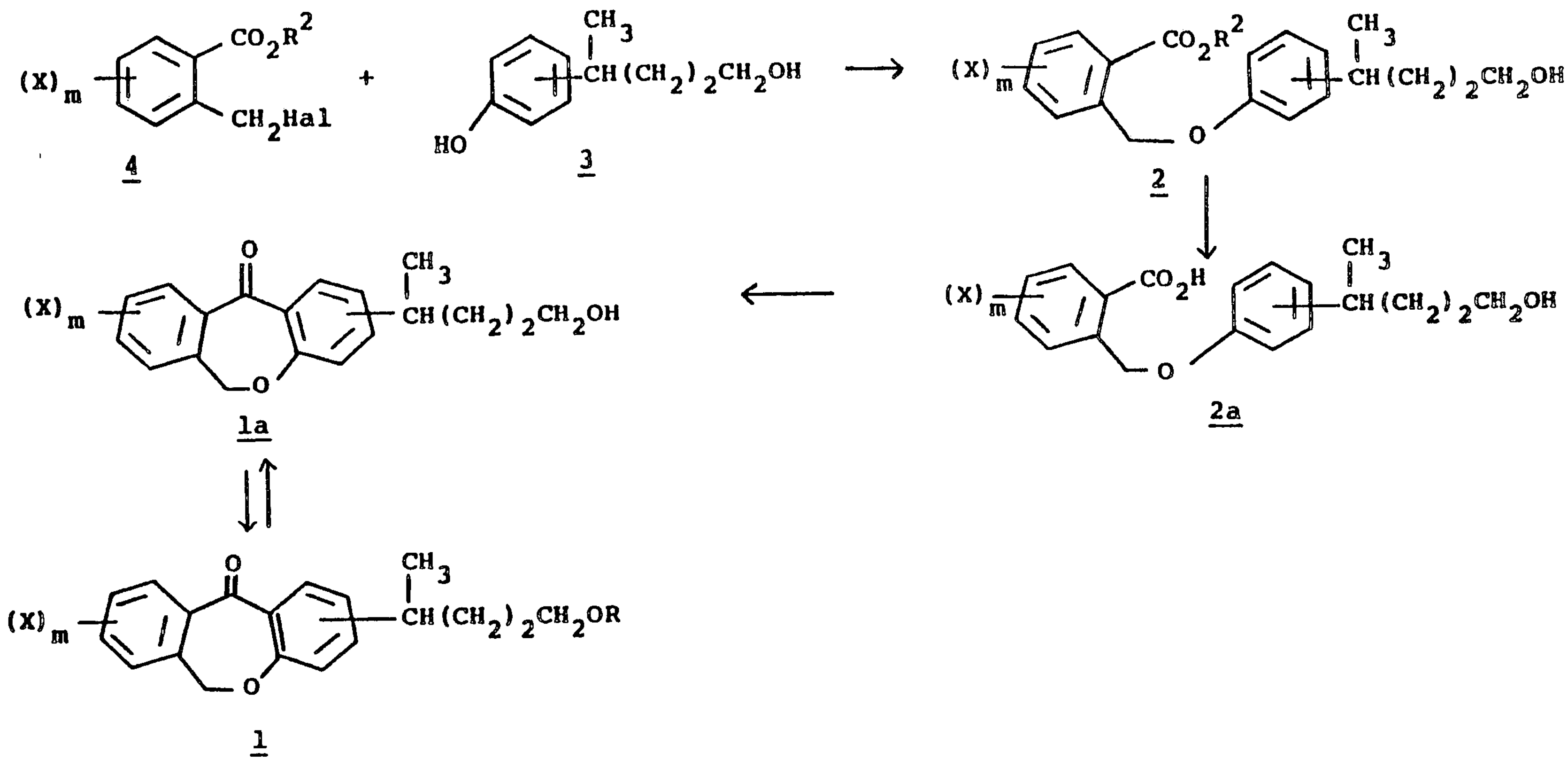

wherein R, Hal, $R^2$, X and m are as hereinbeforedefined.

REACTION SCHEME B

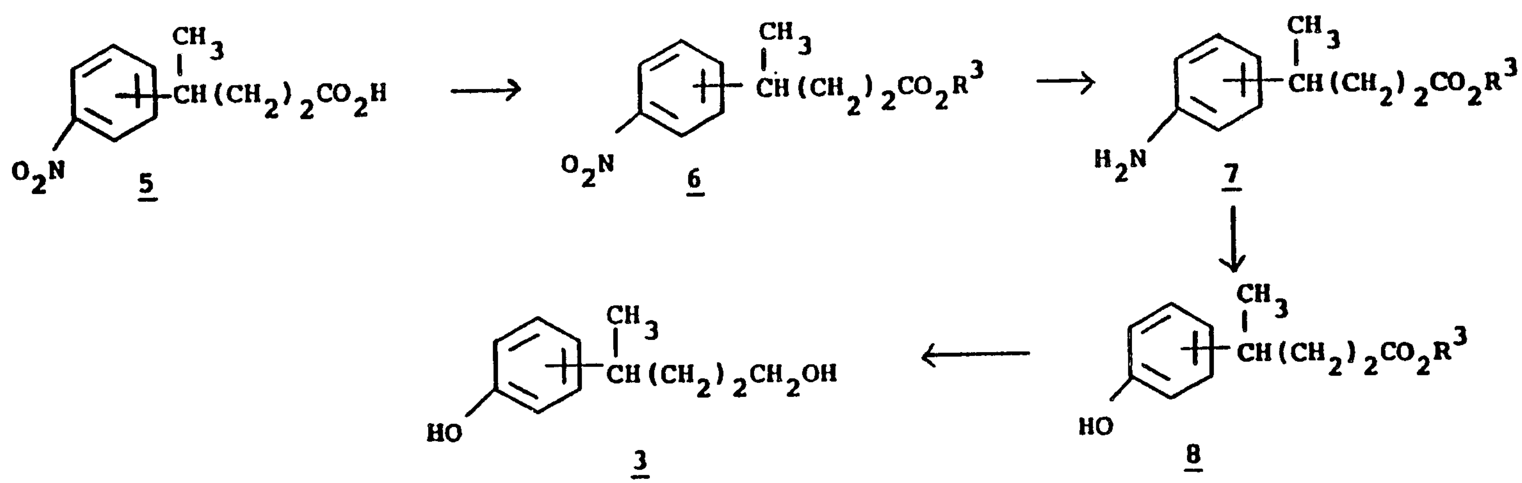

5 6 7 8 3

wherein $R^3$ is as hereinbeforedefined.

The following examples are for illustration purposes only and are not to be construed as limiting the invention. All temperatures are given in degrees centigrade (°C).

Example 1

4-(4-Hydroxyphenyl)pentanol

A solution of 30 g of ethyl 4(4-hydroxyphenyl)pentanoate in 150 ml of toluene (sieve dried), under an atmosphere of nitrogen, was maintained at a temperature between 20—25°C, while 270 ml of 1M borane methyl sulfide/dichloromethane was added dropwise over 15 mins. The mixture was heated under reflux overnight, cooled, 200 ml of cold methanol was added cautiously, and nitrogen was bubbled through the mixture for an hour. The mixture was evaporated and the residue was partitioned between ether and water. The ether extract was separated, dried over anhydrous sodium sulfate, filtered and evaporated. Chromatography of the residue on a Waters Associates Prep LC-System 500 (silica gel, 60% hexane: 40% ethyl acetate) gave 13.7 g (57%) of product, as an oil.

Analysis:

| | | |
|---|---|---|
| Calculated for $C_{11}H_{16}O_2$: | 73.30% C | 8.95% H |
| Found: | 72.96% C | 8.80% H |

Example 2

4-[4-(2-Ethoxycarbonylbenzyloxy)phenyl]pentanol

A mixture of 10.38 g of 4-(4-hydroxyphenyl)pentanol, 15.73 g of ethyl-bromo-o-toluate, 31.79 g of anhydrous potassium carbonate and 0.5 g of pulverised potassium iodide in 250 ml 2-butanone was heated under reflux overnight. The salts were collected and washed with ether. The filtrate was evaporated and the residue was partitioned between water and ether. The ether extract was washed with 10% of sodium hydroxide solution and water, dried over anhydrous sodium sulfate, filtered and evaporated. Chromatography of the residue on a Waters Associates Prep LC-System 500 (silica gel, 7:3 hexane:ethyl acetate) gave 9.98 g (51%) of product, as an oil.

Analysis:

| | | |
|---|---|---|
| Calculated for $C_{21}H_{26}O_4$: | 73.66% C | 7.65% H |
| Found: | 73.93% C | 7.70% H |

Example 3

4-[4-(2-Carboxybenzyloxy)phenyl]pentanol

A mixture of 7.78 g of 4-[4-(2-ethoxycarbonylbenzyloxy)phenyl]pentanol, 19 g of potassium hydroxide in 20 ml of water and 125 ml of 95% of ethanol was heated under reflux overnight. The mixture was cooled and evaporated *in vacuo*. The residue was partitioned between water and ether. The aqueous layer was cooled and acidified with conc. hydrochloric acid. The oil, which formed, was extracted with dichloromethane, and the extract was washed with water, dried over anhydrous sodium sulfate, filtered and evaporated. Trituration of the residue with pentane gave 58 g (81%), of product, mp 59—62.5°.

Analysis:

| | | |
|---|---|---|
| Calculated for $C_{19}H_{22}O_4$: | 72.59% C | 8.05% H |
| Found: | 72.33% C | 7.18% H |

Example 4

4-(6,11-Dihydro-11-oxodibenz[b,e]oxepin-2-yl)pentanol

A solution of 8.32 g of 4-[4-(2-carboxybenzyloxy)phenyl]pentanol in 70 ml of dichloromethane was treated with 13.65 g of trifluoroacetic anhydride. The mixture was heated under reflux for 2 hrs and allowed to stand at ambient temperature for two days. The mixture was cooled and treated with 70 ml of water. The organic layer was separated and washed with 5% of sodium bicarbonate solution, dried over anhydrous sodium sulfate, filtered, and evaporated. The residue was dissolved in a mixture of 150 ml of acetone and 125 ml of 5% of hydrochloric acid and refluxed for 2 hrs. After standing overnight, the acetone was evaporated and the residual aqueous layer was extracted with ether. The ether extract was washed with 5% of sodium bicarbonate solution, with water until neutral, dried over anhydrous sodium sulfate, filtered and evaporated. Chromatography on a Waters Associates Prep LC-System 500 (silica gel, hexane:ethyl acetate 6:4) gave 4.24 g (58%) of product, as an oil.

Analysis:

| | | |
|---|---|---|
| Calculated for $C_{19}H_{20}O_3$: | 77.00% C | 6.80% H |
| Found: | 76.65% C | 6.90% H |

**Claims for the Contracting States: BE CH DE FR GB IT LI LU NL SE**

1. A compound of the formula *1*

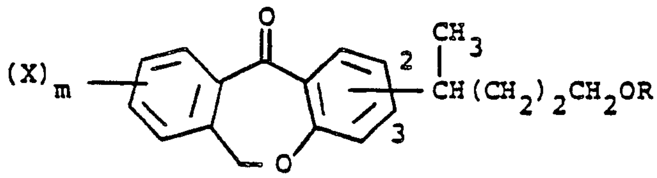

1

wherein the group

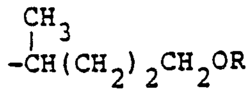

is bound to either the 2- or 3-position and wherein R is hydrogen or a group of the formula

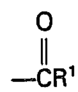

wherein $R^1$ is hydrogen, alkyl of 1 to 5 carbon atoms, benzyl, phenyl or phenyl substituted by alkyl of 1 to 5 carbon atoms, alkoxy of 1 to 5 carbon atoms, halogen or trifluoromethyl; X is alkyl of 1 to 5 carbon atoms, alkoxy of 1 to 5 carbon atoms, halogen or trifluoromethyl; m is 0, 1 or 2; or an optical antipode thereof.

2. A compound according to claim 1 wherein R is hydrogen, and m is 0.

3. The compound according to claim 2 which is 4-(6,11-dihydro-11-oxodibenz[b,e]oxepin-2-yl)pentanol.

4. A pharmaceutical composition comprising as active ingredient a compound as claimed in claim 1 in association with a pharmaceutically acceptable carrier.

5. Use of a compound as claimed in claim 1 for the preparation of a medicament having antiarthritic properties.

6. A compound of the formula *2*

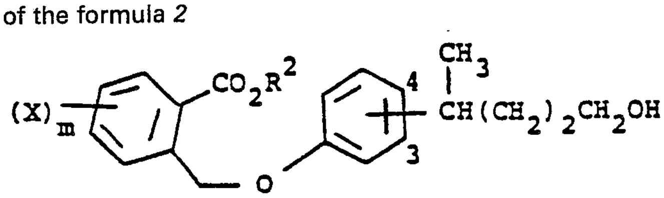

2

wherein the group

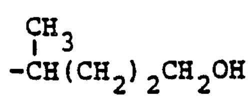

is bound to either the 3- or 4-position wherein $R^2$ is hydrogen or alkyl of 1 to 5 carbon atoms; X is alkyl of 1 to 5 carbon atoms; alkoxy of 1 to 5 carbon atoms, halogen or trifluoromethyl; m is 0, 1 or 2, or an optical antipode thereof.

7. A process for the preparation of a compound as defined in claim 1 which comprises cyclizing a compound of the formula *2*

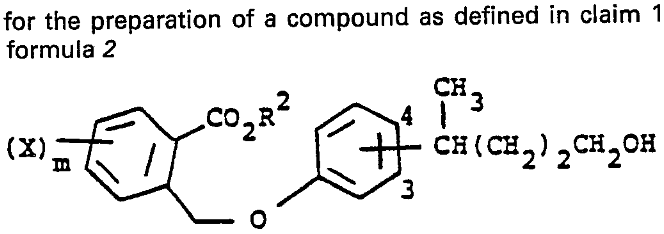

2

wherein the group

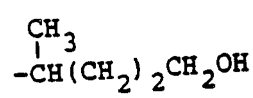

is bound to either the 3- or 4-position, and wherein X and m are as defined in claim 1 in the presence of a dehydrating agent, and optionally esterifying the compound of the formula *1* wherein R is hydrogen.

8. A process as claimed in claim 7 wherein the dehydrating agent is trifluoroacetic anhydride, polyphosphoric acid, polyphosphoric acid-alkanoic acid, phosphorus pentoxide or phosphorus pentoxide-alkanol.

9. The process as claimed in claim 7 wherein the cyclization is carried through in the presence of a halocarbon solvent and at a temperature from ambient temperature to the reflux temperature of the reaction medium.

**Claims for the Contracting State: AT**

1. A process for the preparation of a compound of the formula *1*

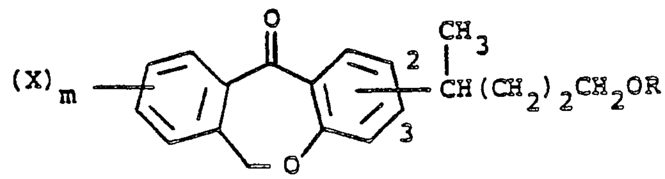

1

wherein the group

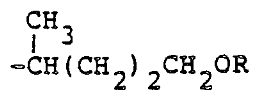

is bound to either the 2- or 3-position and wherein R is hydrogen or a group of the formula

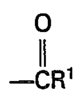

wherein $R^1$ is hydrogen, alkyl of 1 to 5 carbon atoms, benzyl, phenyl or phenyl substituted by alkyl of 1 to 5 carbon atoms, alkoxy of 1 to 5 carbon atoms, halogen or trifluoromethyl; X is alkyl of 1 to 5 carbon atoms, alkoxy of 1 to 5 carbon atoms, halogen or trifluoromethyl; m is 0, 1 or 2; or an optical antipode thereof, which comprises cyclizing a compound of the formula *2*

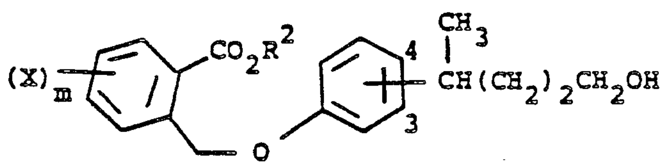

2

wherein the group

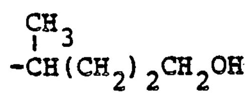

is bound to either the 3- or 4-position and wherein X and m are as defined above, in the presence of a dehydrating agent, and optionally esterifying the compound of the formula *1* wherein R is hydrogen in a usual manner.

2. A process according to claim 1 wherein R is hydrogen, and m is 0.

3. A process as claimed in claim 2 wherein the compound 4-(6,11-dihydro-11-oxodibenz[b,e]oxepin-2-yl)pentanol is prepared.

4. A process as claimed in any claims 1 to 3 wherein the cyclization is carried through in the presence of trifluoroacetic anhydride, polyphosphoric acid, polyphosphoric acid-alkanoic acid, phosphorous pentoxide or phosphorous pentoxide-alkanol, as dehydrating agent, in a halocarbon solvent and at a temperature from ambient to the reflux temperature of the reaction medium.

5. A process for the preparation of a compound of the formula *2*

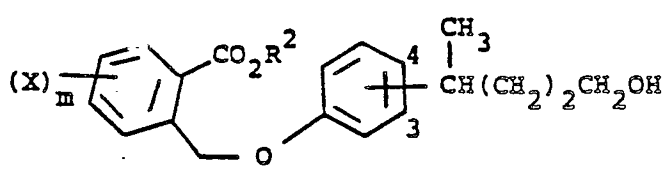

2

wherein the group

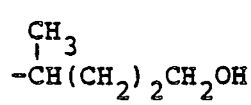

is bound to either the 3- or 4-position, and wherein $R^2$ is alkyl of 1 to 5 carbon atoms; X is alkyl of 1 to 5 carbon atoms, loweralkoxy of 1 to 5 carbon atoms, halogen or trifluoromethyl; m is 0, 1 or 2, or the optical antipode thereof, which comprises reacting a compound of the formula *4*

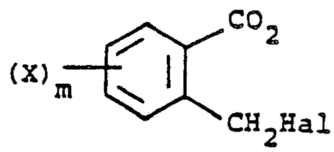

4

wherein $R^2$, X and m are as above; and Hal is halogen, with a compound of the formula *3*

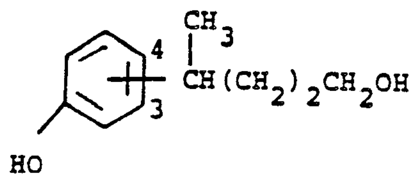

3

wherein the group

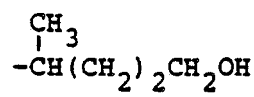

is bound to either the 3- or 4-position, or an optical antipode thereof, in an alkanone in the presence of an alkali metal carbonate.

**Patentansprüche für die Vertragsstaaten: BE CH DE FR GB IT LI LU NL SE**

1. Eine Verbindung der Formel *1*

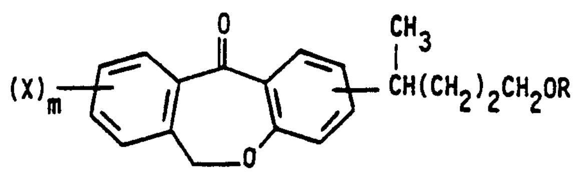

1,

wobei die Gruppe

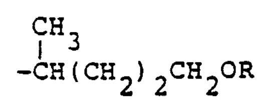

entweder an die 2- oder an die 3-Stellung gebunden ist, und worin R Wasserstoff oder eine Gruppe der Formel

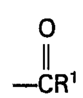

ist, wobei $R^1$ Wasserstoff, Alkyl mit 1 bis 5 Kohlenstoffatomen, Benzyl, Phenyl oder durch Alkyl mit 1 bis 5 Kohlenstoffatomen, Alkoxy mit 1 bis 5 Kohlenstoffatomen, Halogen oder Trifluormethyl substituiertes Phenyl ist; X Alkyl mit 1 bis 5 Kohlenstoffatomen, Alkoxy mit 1 bis 5 Kohlenstoffatomen, Halogen oder Trifluormethyl ist; und m 0, 1 oder 2 ist; oder ein optischer Antipode davon.

2. Eine Verbindung nach Anspruch 1, wobei R Wasserstoff ist, und m 0 ist.

3. Die Verbindung nach Anspruch 2, welche 4-(6,11-Dihydro-11-oxodibenz[b,e]oxepin-2-yl)pentanol ist.

4. Eine pharmazeutische Zusammensetzung, enthaltend als Wirkbestandteil eine wie in Anspruch 1 beanspruchte Verbindung gemeinsamen mit einem pharmazeutisch annehmbaren Träger.

5. Verwendung einer wie in Anspruch 1 beanspruchten Verbindung für die Herstellung eines Arzneimittels mit antiarthritischen Eigenschaften.

6. Eine Verbindung der Formel *2*

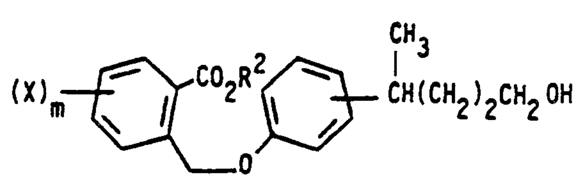

2,

wobei die Gruppe

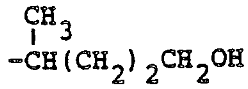

entweder an die 3- oder an die 4-Stellung gebunden ist, und worin $R^2$ Wasserstoff oder Alkyl mit 1 bis 5 Kohlenstoffatomen ist; X Alkyl mit 1 bis 5 Kohlenstoffatomen, Alkoxy mit 1 bis 5 Kohlenstoffatomen, Halogen oder Trifluormethyl ist; m 0, 1 oder 2 ist; oder ein optischer Antipode davon.

7. Ein Verfahren zur Herstellung einer wie in Anspruch 1 definierten Verbindung, welches das Cyclisieren einer Verbindung der Formel *2*

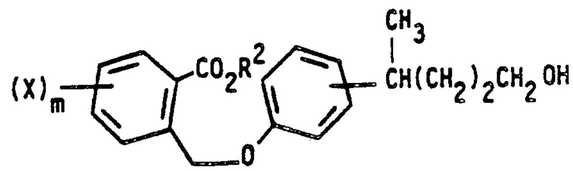

2 ,

wobei die Gruppe

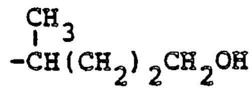

entweder an die 3- oder an die 4-Stellung gebunden ist, und worin X und m wie in Anspruch 1 definiert sind, in Gegenwart eines Dehydratisierungsmittels, und gegebenenfalls das Verestern der Verbindung der Formel *1*, worin R Wasserstoff ist, umfaßt.

8. Das Verfahren wie in Anspruch 7 beansprucht, wobei das Dehydratisierungsmittel Trifluoressigsäureanhydrid, Polyphosphorsäure, Polyphosphorsäure-Alkansäure, Phosphorpentoxid oder Phosphorpentoxid-Alkanol ist.

9. Das Verfahren wie in Anspruch 7 beansprucht, wobei die Cyclisierung in Gegenwart eines Halogenkohlenstofflösungsmittels und bei einer Temperatur von Umgebungstemperatur bis zur Rückflußtemperatur des Reaktionsmediums durchgeführt wird.

**Patentansprüche für den Vertragsstaat: AT**

1. Ein Verfahren zur Herstellung einer Verbindung der Formel *1*

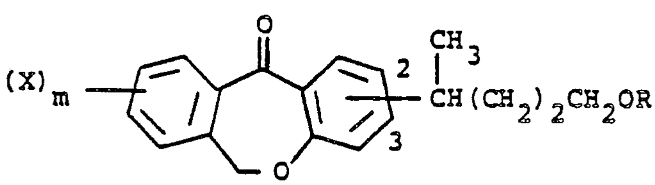

1

wobei die Gruppe

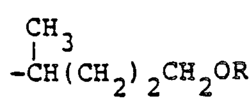

entweder an die 2- oder an die 3-Stellung gebunden ist, und worin R Wasserstoff oder eine Gruppe der Formel

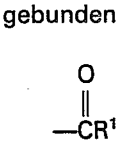

ist, wobei $R^1$ Wasserstoff, Alkyl mit 1 bis 5 Kohlenstoffatomen, Benzyl, Phenyl oder durch Alkyl mit 1 bis 5 Kohlenstoffatomen, Alkoxy mit 1 bis 5 Kohlenstoffatomen, Halogen oder Trifluormethyl substituiertes Phenyl ist; X Alkyl mit 1 bis 5 Kohlenstoffatomen, Alkoxy mit 1 bis 5 Kohlenstoffatomen, Halogen oder Trifluormethyl ist; und m 0, 1 oder 2 ist; oder eines optischen Antipoden davon, welches das in einer üblichen Weise erfolgende Cyclisieren einer Verbindung der Formel *2*

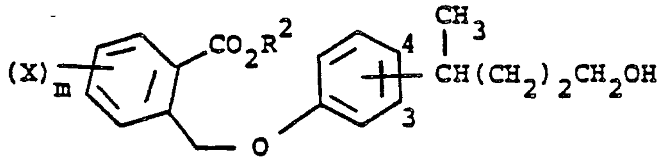

2

wobei die Gruppe

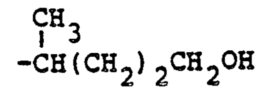

entweder an die 3- oder an die 4-Stellung gebunden ist, und worin X und m wie oben definiert sind, in Gegenwart eines Dehydratisierungsmittels, und gegebenenfalls das in einer üblichen Weise erfolgende Verestern der Verbindung der Formel *1*, worin R Wasserstoff ist, umfaßt.

2. Ein Verfahren nach Anspruch 1, wobei R Wasserstoff ist, und m 0 ist.

3. Ein Verfahren nach Anspruch 2, bei dem die Verbindung 4-(6,11-Dihydro-11-oxodibenz[b,e]oxepin-2-yl)pentanol hergestellt wird.

4. Ein Verfahren wie in einem der Ansprüche 1 bis 3 beansprucht, wobei die Cyclisierung in Gegenwart von Trifluoressigsäureanhydrid, Polyphosphorsäure, Polyphosphorsäure-Alkansäure, Phosphorpentoxid oder Phosphorpentoxid-Alkanol als Dehydratisierungsmittel, in einem Halogenkohlenstofflösungsmittel und bei einer Temperatur von Umgebungstemperatur bis zur Rückflußtemperatur des Reaktionsmediums durchgeführt wird.

5. Ein Verfahren zur Herstellung einer Verbindung der Formel *2*

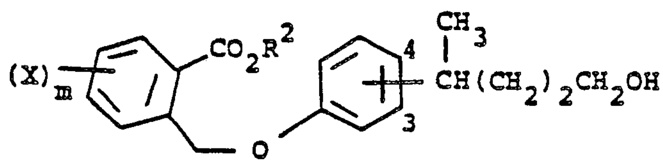

2

wobei die Gruppe

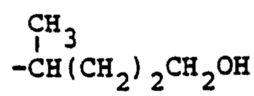

entweder an die 3- oder an die 4-Stellung gebunden ist, und worin $R^2$ Alkyl mit 1 bis 5 Kohlenstoffatomen ist; X Alkyl mit 1 bis 5 Kohlenstoffatomen, Niedrigalkoxy mit 1 bis 5 Kohlenstoffatomen, Halogen oder Trifluormethyl ist; m 0, 1 oder 2 ist; oder des optischen Antipoden davon, welches das Umsetzen einer Verbindung der Formel *4* .

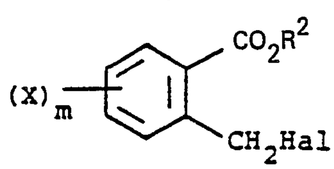

4

worin $R^2$, X und m wie oben definiert sind, und Hal Halogen ist, mit einer Verbindung der Formel *3*

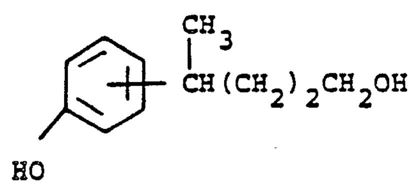

3

worin die Gruppe

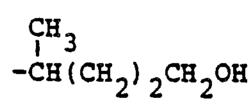

entweder an die 3- oder an die 4-Stellung gebunden ist, oder eines optischen Antipoden davon, in einem Alkanon in Gegenwart eines Alkalimetallcarbonats umfaßt.

**Revendications pour les Etats contractants: BE CH DE FR GB IT LI LU NL SE**

1. Composé de formule *1*:

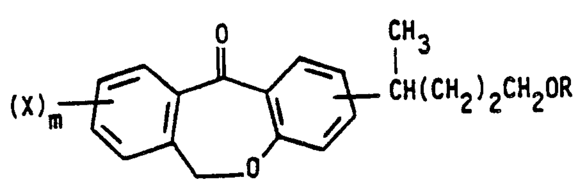

1,

dans laquelle le groupe

$$-\underset{|}{\overset{CH_3}{}}CH(CH_2)_2CH_2OR$$

est lié au niveau de la position 2- ou 3-, et dans laquelle R représente un atome d'hydrogène ou un groupe de formule

$$\overset{O}{\overset{\|}{C}}R^1$$

dans laquelle $R^1$ représente un atome d'hydrogène, un groupe alkyle comportant de 1 à 5 atomes de carbone, un groupe benzyle, phényle ou phényle substitué avec un groupe alkyle comportant de 1 à 5 atomes de carbone, un groupe alkoxy comportant de 1 à 5 atomes de carbone, un atome d'halogène ou un groupe trifluorométhyle; X représente un groupe alkyle comportant de 1 à 5 atomes de carbone, un groupe alkoxy comportant de 1 à 5 atomes de carbone, un atome d'halogène ou un groupe trifluorométhyle; m est égal à 0, 1 ou 2; ou un énantiomère de celui-ci.

2. Composé selon la revendication 1, dans lequel R représente un atome d'hydrogène, et m est égal à 0.

3. Composé selon la revendication 2, qui est le 4-(6,11-dihydro-11-oxodibenzo[b,e]oxépin-2-yl)pentanol.

4. Composition pharmaceutique, comprenant, comme ingrédient actif, un composé selon la revendication 1, associé à un véhicule pharmaceutiquement acceptable.

5. Utilisation d'un composé selon la revendication 1 pour la préparation d'un médicament ayant des propriétés anti-arthritiques.

6. Composé de formule *2*:

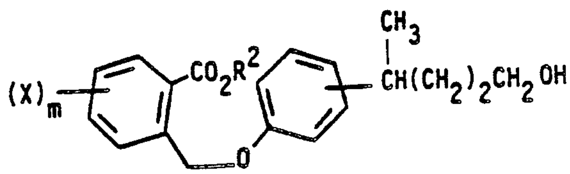

2,

dans laquelle le groupe

$$-\underset{|}{\overset{CH_3}{}}CH(CH_2)_2CH_2OH$$

est lié au niveau de la position 3- ou 4, et dans laquelle $R^2$ représente un atome d'hydrogène ou un groupe alkyle comportant de 1 à 5 atomes de carbone; X représente un groupe alkyle comportant de 1 à 5 atomes de carbone, un groupe alkoxy comportant de 1 à 5 atomes de carbone, un atome d'halogène ou un groupe trifluorométhyle; m est égal à 0, 1 ou 2; ou un énantiomère de celui-ci.

7. Procédé de préparation d'un composé selon la revendication 1, dans lequel on cyclise un composé de formule *2*:

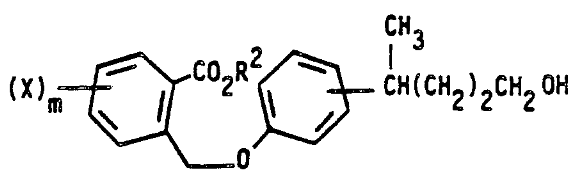

2,

dans laquelle le groupe

$$-\underset{|}{\overset{CH_3}{}}CH(CH_2)_2CH_2OH$$

est lié au niveau de la position 3- ou 4-, et dans laquelle X et m sont tels que définis dans la revendication 1, en présence d'un agent déshydratant, et on estérifie éventuellement le composé de formule I dans laquelle R représente un atome d'hydrogène.

8. Procédé selon la revendication 7, dans lequel l'agent déshydratant, est l'anhydride trifluoroacétique, l'acide polyphosphorique, l'acide polyphosphorique associé à une acide alcanoïque, le pentoxyde de phosphore ou le pentoxyde de phosphore associé à un alcanol.

9. Procédé selon la revendication 7, dans lequel la cyclisation est effectuée en présence d'un solvant halocarboné et à une température allant de la température ambiante à la température de reflux du milieu réactionnel.

**Revendications pour l'Etat contractant: AT**

1. Procédé de préparation d'un composé de formule *1*:

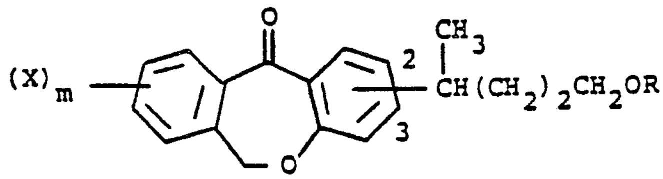

1

dans laquelle le groupe

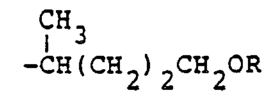

est lié au niveau de la position 2- ou 3-, et dans laquelle R représente un atome d'hydrogène ou un groupe de formule

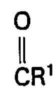

dans laquelle $R^1$ représente un atome d'hydrogène, un groupe alkyle comportant de 1 à 5 atomes de carbone, un groupe benzyle, phényle ou phényle substitué avec un groupe alkyle comportant de 1 à 5 atomes de carbone, un groupe alkoxy comportant de 1 à 5 atomes de carbone, un atome d'halogène ou un groupe trifluorométhyle; X représente un groupe alkyle comportant de 1 à 5 atomes de carbone, un groupe alkoxy comportant de 1 à 5 atomes de carbone, un atome d'halogène ou un groupe trifluorométhyle; m est égal à 0, 1 ou 2; ou un énantiomère de celui-ci, dans lequel on cyclise un composé de formule *2*:

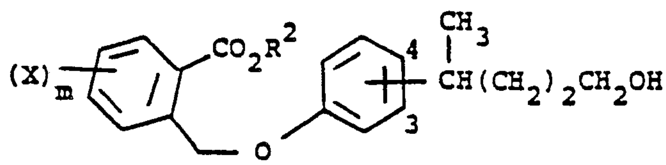

2

dans laquelle le groupe

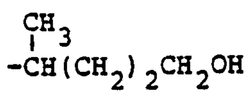

est lié au niveau de la position 3- ou 4-, et dans laquelle X et m sont tels que définis ci-dessus, en présence d'un agent déshydratant, et on estérifie éventuellement, de façon classique, le composé de formule I dans laquelle R représente un atome d'hydrogène.

2. Procédé selon la revendication 1, dans lequel R représente un atome d'hydrogène et m est égal à 0.

3. Procédé selon la revendication 2, dans lequel le composé préparé est le 4-(6,11-dihydro-11-oxodibenzo[b,e]oxépin-2-yl)pentanol.

4. Procédé selon l'une quelconque des revendications 1 à 3, dans lequel la cyclisation est effectuée en présence d'anhydride trifluoroacétique, d'acide polyphosphorique, d'acide polyphosphorique associé à un acide alcanoïque, de pentoxyde de phosphore ou de pentoxyde de phosphore associé à un alcanol, comme agent déshydratant, dans un solvant halocarboné et à une température allant de la température ambiante à la température de reflux du milieu réactionnel.

5. Procédé de préparation d'un composé de formule *2*:

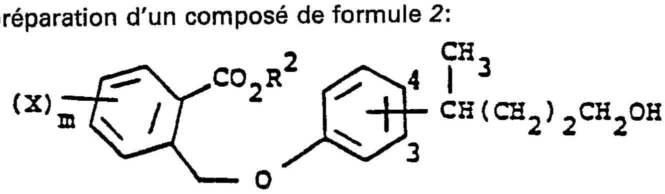

2

dans laquelle le groupe

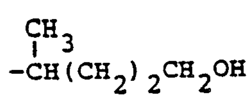

est lié au niveau de la position 3- ou 4-, et dans laquelle $R^2$ représente un groupe alkyle comportant de 1 à 5 atomes de carbone, X représente un groupe alkyle comportant de 1 à 5 atomes de carbone, un groupe

alkoxy inférieur comportant de 1 à 5 atomes de carbone, un atome d'halogène ou un groupe trifluorométhyle; m est égal à 0, 1 ou 2, ou l'énantiomère de celui-ci, dans lequel on fait réagir un composé de formule *4*:

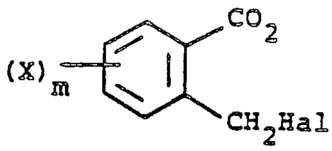

4

dans laquelle $R^2$, X et m sont tels que définis ci-dessus; et Hal représente un atome d'halogène, avec un composé de formule *3*:

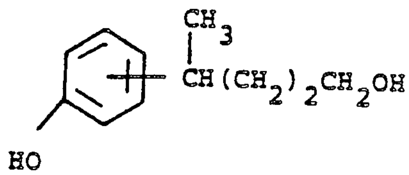

3

dans laquelle le groupe

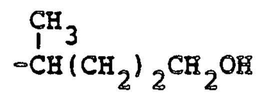

est lié au niveau de la position 3- ou 4-, ou un énantiomère de celui-ci, dans une alcanone en présence d'un carbonate de métal alcalin.